# EUROPEAN PATENT SPECIFICATION

(11) **EP 1 800 607 B1**
(45) Date of publication and mention of the grant of the patent: **18.02.2009**
(21) Application number: 06025444.8
(22) Date of filing: 08.12.2006
(51) Int. Cl.: A61B 17/04, A61B 17/06

(54) **Reinforcing tool for a medical needle**
Werkzeug mit Verstärkung für eine medizinische Nadel
Outil de renforcement d'une aiguille médicale

(30) Priority: 26.12.2005 JP 2005371422
(43) Date of publication of application: 27.06.2007
(73) Proprietor: Covidien AG, 8212 Neuhausen am Rheinfall (CH)
(72) Inventor: Abe, Kazuhiro, Fukuroi-shi Shizuoka-ken (JP); Fujiwara, Norifumi, Fukuroi-shi Shizuoka-ken (JP)
(74) Representative: Tomlinson, Edward James

(56) References cited:
- WO-A-02/065921
- WO-A-03/013392
- DE-A1- 10 245 076
- DE-U1- 20 109 379
- US-A- 5 405 353
- US-A1- 2005 101 984
- US-B1- 6 325 807

## Description

### Technical field

The present invention relates to a detachable reinforcing tool for a medical needle that is attached to a medical needle used to insert a drain tube into a wound after surgery.

### Background art

In the past, a drain tube would be inserted into the wound after a surgical procedure to drain bodily secretions produced in the wound. In this case, the part at the back end of the drain tube would be kept in the wound by attaching a medical needle to the leading end of the drain tube and passing the medical needle into the patient's body (e.g. Japanese Patent Application No. JP 60-7851 [1985]).

### Summary of the invention

However, because the abovementioned medical needles are very strong, they are difficult to bend, and this causes problems when it is necessary to use them after being bent from the original state. There is also the problem that medical needles are difficult to handle when they are pulled from the body, since they are slippery. For this reason, there are also medical needles that have a depression or narrow diameter part in the middle to make bending easy as well as making them easy to hold. However, the strength is insufficient because of this, and there is the risk that the medical needle will bend unintentionally when a strong force is applied. An example of a needle with a depression or narrow diameter part to make bending easy is described in US 2005/0101984 A1, with alternatives for providing for bending described therein being the use of different materials and particular material treatment regimes. Examples of needles with a slotted or recessed part in which the slot or recess is not provided to make bending easy include WO 03/013392 A1 and DE 201 09 379 U1, both of which describe arrangements for attaching a handle to the needle. In WO 02/065921 A1, a needle arrangement is described in which a handle is attached by a gripping screw after the needle has been inserted into a slot in the handle.

The present invention was devised taking this situation into consideration. Its objective is to provide a reinforcing tool for a medical needle with which the medical needle can be bent as necessary, and that can also increase the strength.

In order to accomplish the aforementioned objective, the features in the constitution of the reinforcing tool for a medical needle pertaining to the present invention consist in that it is a reinforcing tool for attachment to a medical needle on which a connector for connecting a drain tube to be put into a wound area after surgery is formed at the back end, a puncturing part is formed at the tip, and a narrow diameter part is formed in the portion near the center, which is constituted with a cylindrical body that can be detachably attached to the narrow diameter part of the aforementioned medical needle.

This reinforcing tool is constituted to be attached to the narrow diameter part of the medical needle that has low strength and is easy to bend. Therefore, the strength of the medical needle can be increased by attaching the reinforcing tool to the narrow diameter part of the medical needle and the medical needle can be prevented from bending accidentally during use. When the medical needle is to be used while bent, the narrow diameter part can be bent by removing the reinforcing tool from the medical needle. When the medical needle is to be pulled out of the body, removal of the reinforcing tool from the medical needle when the reinforcing tool leaves the body along with the puncturing part facilitates subsequent handling by holding the narrow diameter part. In addition, a level difference or gap can be provided between the peripheral surface of the main body of the medical needle and the end part of the reinforcing tool when the reinforcing tool is attached to the medical needle. This prevents the hand from slipping when the medical needle is handled.

Another feature in the constitution of the reinforcing tool for a medical needle pertaining to the present invention is that the outer diameter of the reinforcing tool is approximately the same size as the outer diameter of the portion in the center that constitutes the main body of the aforementioned medical needle outside of the aforementioned narrow diameter part. Because of this, the thickness of the portion near the longitudinal center of the medical needle is approximately uniform, so that discomfort to the patent when the medical needle punctures the patient's body can be reduced, and handling will be easier.

Still another feature in the constitution of the reinforcing tool for a medical needle pertaining to the present invention is that the reinforcing tool is constituted with two divided parts that are basically gutter shaped, have semicircular cross sections, and produce a cylindrical shape when the longitudinal edges of the aforementioned two divided parts are joined. Because of this, operations to join and remove the two divided parts from the medical needle will be simple.

Still other features in the constitution of the reinforcing tool for a medical needle pertaining to the present invention are that one of the two divided parts is constituted with elasticity, an engaging part is provided on each of the outside surfaces of the two longitudinal edges of one of the aforementioned divided parts, engageable parts that can engage with the two engaging parts of the aforementioned one divided member are provided on the inside surfaces of the two longitudinal edges of the other of the aforementioned two parts, and the aforementioned one divided member can be attached or detached from the aforementioned other divided member by flexing the longitudinal edges of the aforementioned one member toward the central axis.

Because of this, when the two parts are joined to each other, one divided member can be engaged with the other divided member by releasing the pressure on the two edges of the one divided member after the two edges of the one divided member have been pressed toward the central axis and flexed to position them inside the two edges of the other divided member. In this case, because the one divided member has elasticity, the two edges return to their original state when released from the force pressing them toward the central axis, and the engaging parts of the one divided member engage in the engageable parts of the other divided member. When the two parts that are joined are to be removed, the engaging parts of the one divided member and the engageable parts of the other divided member can be disengaged by pressing the two edges of the one divided member toward the central axis to flex them. Then the two parts can be disassembled by separating one divided member from the other divided member.

Still another feature in the constitution of the reinforcing tool for a medical needle pertaining to the present invention is that the medical needle is used by bending the narrow diameter part at a prescribed angle and the reinforcing tool is bent in advance to match the bending angle of the narrow diameter part. Because of this, the strength of the narrow diameter part of the reinforcing needle is increased when bent, and smooth operation is permitted.

Still another feature in the constitution of the reinforcing tool for a medical needle pertaining to the present invention is that the reinforcing tool is constituted to be bendable. Because of this, when the narrow diameter part of the medical needle is bent at a prescribed angle, the reinforcing tool can also be bent to match the bending angle of the narrow diameter part. It is preferred for the reinforcing tool in this case be harder to bend than the narrow diameter part of the medical needle, so that when the reinforcing tool is attached to the narrow diameter part, the narrow diameter part and the reinforcing tool will not bend because of the combined strength. Because of this, it is possible to bend the medical needle at a prescribed angle [when needed], while the medical needle can also be prevented from bending during actual use in surgery.

### Brief description of the figures

The invention will now be described, by way of example only, with reference to the accompanying drawings in which:
Figure 1 is an oblique view showing a medical needle to which is attached a reinforcing tool pertaining to a first embodiment of the present invention.
Figure 2 shows a medical needle to which a reinforcing tool is attached, with (a) being a side view and (b) a bottom view.
Figure 3 is an oblique view showing a medical needle. Figure 4 shows the reinforcing tool assembled, with (a) being a side view and (b) a front view.
Figure 5 shows the reinforcing tool disassembled, with (a) being a side view and (b) a front view.
Figure 6 is a cross section at 6-6 in Figure 5 (b).
Figure 7 shows a reinforcing tool attached to a medical needle, with (a) being a bottom view and (b) a cross section of Figure 7 (a).
Figure 8 shows the divided parts that constitute the reinforcing tool attached to the medical needle and disengaged, with (a) being a bottom view and (b) a cross section of Figure 8 (a).
Figure 9 shows one of the divided parts that constitute the reinforcing tool attached to the medical needle having been removed, with (a) being a side view and (b) a cross section of Figure 9 (a).
Figure 10 is a side view showing a medical needle with a reinforcing tool removed.
Figure 11 is a side view showing a medical needle to which is attached a reinforcing tool pertaining to a second embodiment.
Figure 12 is a bottom view showing a medical needle to which is attached a reinforcing tool pertaining to a second embodiment.

### Preferred embodiments of the invention

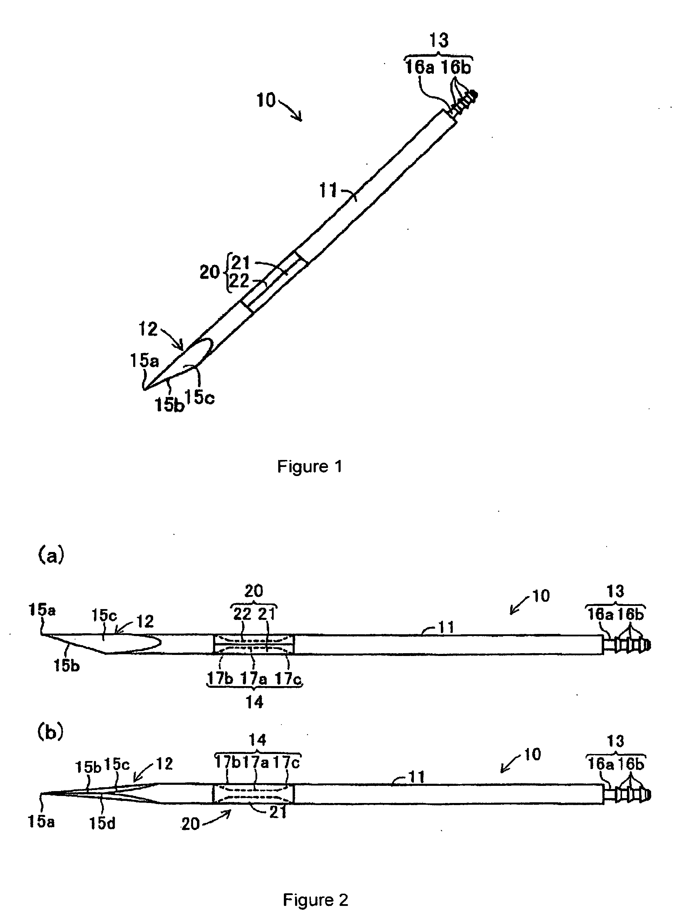

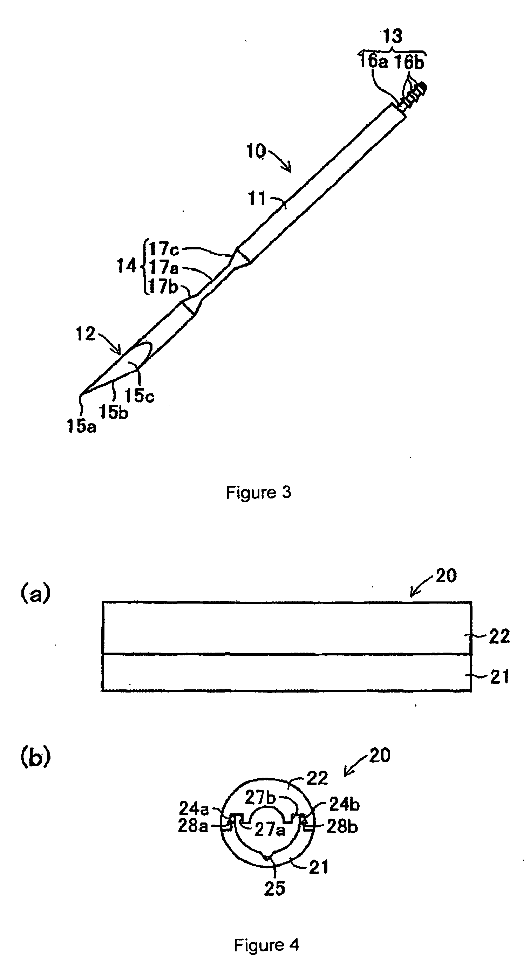

A first embodiment of the present invention will be explained in detail below using the figures. Figures 1 and 2 show medical needle (10) made of metal to which a reinforcing tool (20) pertaining to this embodiment is attached. Medical needle (10) is constituted with a small-diameter circular cylindrical main body (11), a puncturing part (12) formed at the tip of main body (11), and with a connector (13) connected to the rear end of main body (11). A narrow diameter part (14) as shown in Figure 3 is formed at the portion toward the tip in the lengthwise direction of main body (11). Puncturing part (12) is constituted with a needle-shaped body on which flat side surfaces (15c) and (15d) are formed on the two sides such that the top part of the tip is formed into a pointed apex (15a), as shown in Figure 2, and an angled linear part (15b) is formed running from apex (15a) downward and toward the back. In the following, the end with puncturing part (12) will be described as the front, the end with connector (13) as the back, the side of angled linear part (15b) running toward apex (15a) in Figure 2 (a) as the top, and the side opposite from the angled linear part (15b) running from apex (15a) as the bottom.

Connector (13) serves to connect medical needle (10) to a drain tube (not shown) and is constituted with a rod-shaped part (16a) with a smaller diameter than main body (11), and three securing projections (16b) formed at intervals in the lengthwise direction on the periphery of rod-shaped part (16a). The securing parts (16b) are constituted as annular ridges that taper on the outer peripheral surface so that their diameter gradually becomes larger from front to back. For this reason, when a drain tube is to be connected to medical needle (10), it is pressed from the back onto connector (13) so that connector (13) is covered, the inner surface of the drain tube slides forward over the tapered surface of securing projections (16b), and the drain tube becomes connected to connector (13).

Once the drain tube is connected to connector (13), the front peripheral parts of securing projections (16b) provide resistance that makes it difficult for the drain tube to separate from connector (13). Narrow diameter part (14) is constituted with a rod-shaped flexible part (17a), formed to approximately the same thickness as rod-shaped part (16a) of connector (13), and tapered parts (17b) and (17c) formed at the two ends of flexible part (17a). Tapered part (17b) is made so that its diameter gradually becomes larger going forward from the front end of flexible part (17a), and tapered part (17c) is made so that its diameter gradually becomes larger going backward from the back end of flexible part (17a).

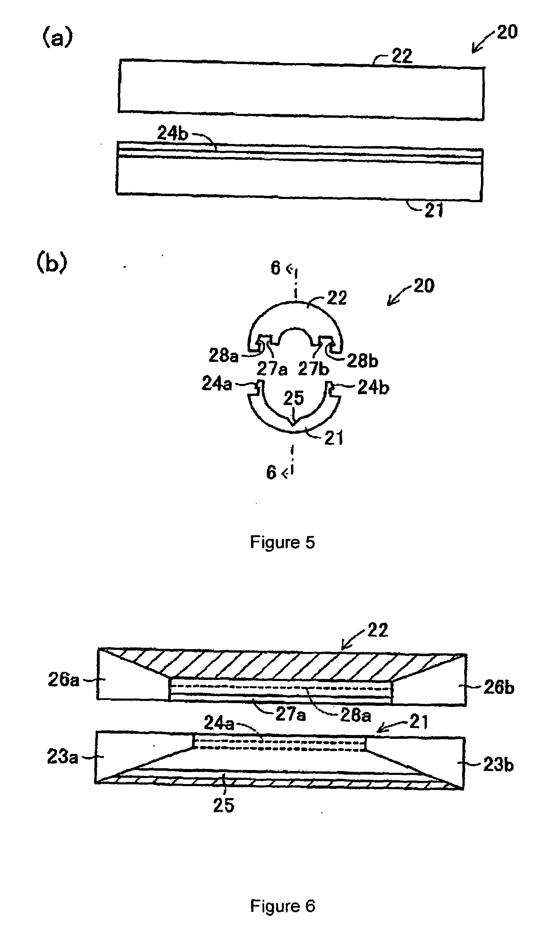

Reinforcing tool (20), as shown in Figures 4 and 5, is constituted with two divided parts (21) and (22) that are basically gutter shaped and can be attached and detached from each other. Both divided parts (21) and (22) are made of stainless steel. The diameter of the outer peripheral surface of divided member (21) (the diameter from left to right in Figures 4 (b) and Figure 5 (b)) is set to approximately the same diameter as main body (11) of medical needle (10), and the diameter of the inner peripheral surface of divided member (21) is set to be larger than the diameter of flexible part (17a) of medical needle (10). For this reason, when reinforcing tool (20) is attached to narrow diameter part (14) of medical needle (10), the outer peripheral surface of medical needle (10) and the outer peripheral surface of divided member (21) lie approximately in the same plane, and a gap of a prescribed size is formed between the inner peripheral surface of divided member (21) and the outer peripheral surface of flexible part (17a).

Also, as shown in Figure 6, tapered recesses (23a) and (23b) that can respectively follow tapered parts (17b) and (17c), at a prescribed spacing from tapered parts (17b) and (17c), of narrow diameter part (14) are formed in the parts at both ends of the inner peripheral surface of divided member (21). Both edge parts following the lengthwise orientation of divided member (21) are made thin by virtue of a portion on the outside surface of each being cut away, and engaging parts (24a) and (24b) composed of projecting lines are formed in the center in the vertical orientation on the outer surface. A notched groove (25) that extends in the lengthwise direction of divided member (21) is also formed in the lower part of the inner surface of divided member (21). Notched groove (25) allows the side portions at both lengthwise edges of divided member (21) to have elasticity in a direction perpendicular to the lengthwise direction. For this reason, divided member (21) can be flexed centered on notched groove (25) by pressing both side parts toward the central axis, and will return to its original state due to its elasticity when the pressure is released.

The outer peripheral surface of divided member (22) is formed to approximately the same size as the outer peripheral surface of divided member (21) and the diameter of the inner peripheral surface of divided member (22) is set to be the same as or slightly larger than the diameter of flexible part (17a) of medical needle (10). For this reason, when reinforcing tool (20) is attached to narrow diameter part (14) of medical needle (10), the outer peripheral surface of medical needle (10) and the outer peripheral surface of divided member (22) will be positioned approximately in the same plane, and the inner peripheral surface of divided member (22) and the outer peripheral surface of flexible part (17a) will nearly touch. Also, as shown in Figure 6, tapered recesses (26a) and (26b), which can respectively follow tapered parts (17b) and (17c) when touching tapered parts (17b) and (17c) of narrow diameter part (14), are formed at the two side parts on the inner circumferential surface of divided member (22).

Recesses (27a) and (27b) are formed in the bottom surfaces of the two longitudinal edges of divided member (22), and engagement recesses (28a) and (28b) that can engage with engaging parts (24a) and (24b) of divided member (21) are formed in the surface positioned to the outside of the two recesses (27a) and (27b). For this reason, when divided member (21) is joined to divided member (22), first, engaging parts (24a) and (24b) of divided member (21) are matched to engagement recesses (28a) and (28b) of divided member (22) with the two edges of divided member (21) being pressed toward the central axis to flex divided member (21).

Then, engaging parts (24a) and (24b) are engaged in engageable parts (28a) and (28b) by releasing the pressure to return divided member (21) to its original state, joining divided member (21) to divided member (22). The two parts (21) and (22) then appear as shown in Figure 4. When divided member (21) is to be removed from divided member (22), divided member (21) is separated from divided member (22) while the two edges of divided member (21) are pressed toward the central axis to flex divided member (21). Then the two divided parts (21) and (22) will be as shown in Figure 5.

In this constitution, when a drain tube is to be inserted into a patient's wound (not shown), reinforcing tool (20) is first attached to narrow diameter part (14) of medical needle (10). Next, the tip of the drain tube is connected to connector (13) of medical needle (10). Next, after medical needle (10) is inserted toward the wound from the incision in the patients body made by puncturing part (12), puncturing part (12) is removed from the body at a specific part of the body. Then, when the portion where reinforcing tool (20) is attached to medical needle (10) appears outside the body, reinforcing tool (20) is removed from medical needle (10), narrow diameter part (14) is gripped with the hand, and medical needle (10) is pulled out of the body.

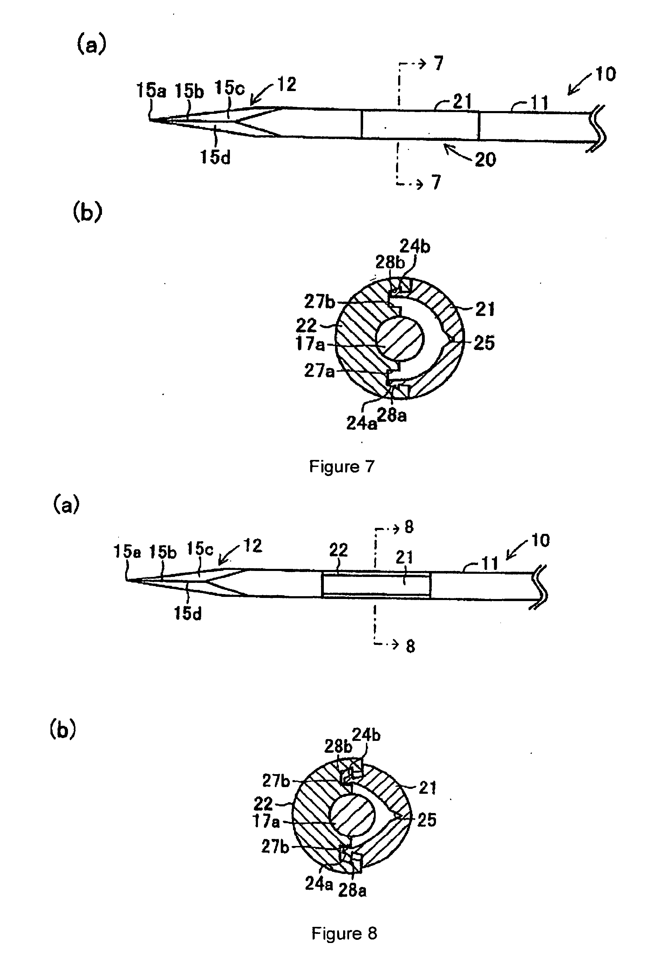

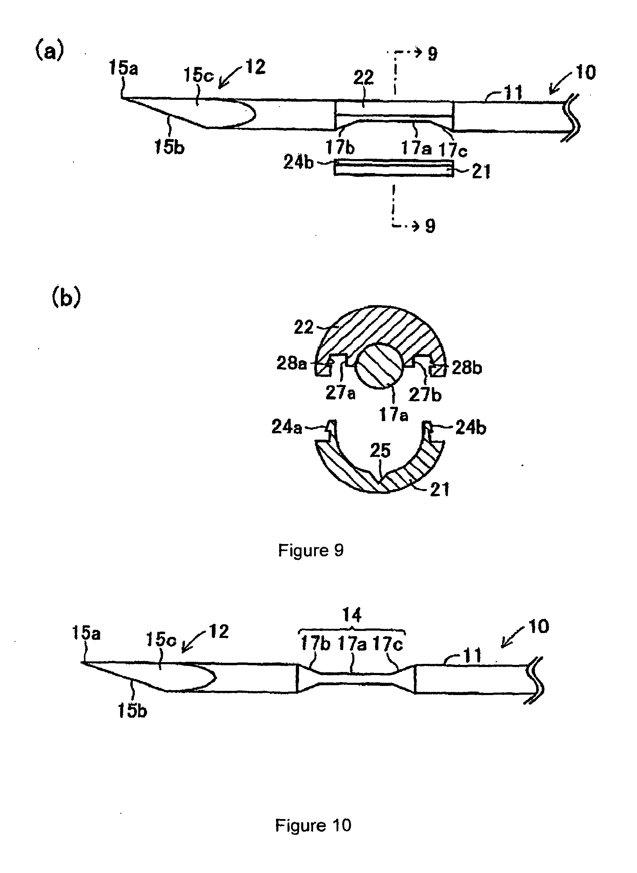

In this case, divided member (21) is flexed to produce the state shown in Figure 8 by squeezing the two edges of divided member (21) toward the inside from the state in which reinforcing tool (20) is attached to narrow diameter part (14) shown in Figure 7. Engaging parts (24a) and (24b) of divided member (21) and engageable parts (28a) and (28b) of divided member (22) are disengaged in this way. Next, divided member (21) is separated from divided member (22) and medical needle (10) to give the state shown in Figure 9. Then the state shown in Figure 10 can be provided by separating divided member (22) from medical needle (10). When medical needle (10) punctures the patient's body, the strength of medical needle (10) is increased since reinforcing tool (20) is attached to narrow diameter part (14) of medical needle (10), and even if greater force is applied during handling, medical needle (10) will not bend accidentally. This makes smooth operation possible.

Then, when the part at the tip of the drain tube exits the body and the back end of the drain tube is positioned in the wound, the pulling operation is stopped and the portion where the drain tube is connected to medical needle (10) is cut with scissors. Next, the part of the drain tube that was cut is connected to a prescribed suction device (not shown). Secretions produced in the wound can then be sucked out through the drain tube by activating the suction device. And when medical needle (10) is to be used bent, reinforcing tool (20) can be separated from medical needle (10) and narrow diameter part (14) can be bent at a specific angle.

In this way, reinforcing tool (20) pertaining to this embodiment is attached to a narrow diameter part (14) in the middle of medical needle (10) that has low strength and bends easily, thus increasing the strength of medical needle (10). Therefore, when a narrow diameter part (14) is provided in medical needle (10) and medical needle (10) is to be used by bending it, while it is possible to bend narrow diameter part (14), the strength of medical needle (10) can be improved by attaching reinforcing tool (20) as needed. And when medical needle (10) is to be pulled out, removing reinforcing tool (20) and holding [the needle by] narrow diameter part (14) keeps the hand from slipping off of medical needle (10).

In addition, since reinforcing tool (20) is constituted with two divided parts (21) and (22) that basically have a gutter shape, their semicircular cross sections can produce a cylindrical shape when divided parts (21) and (22) are joined, and the operations to join and disconnect the two divided parts (21) and (22) will be simple. Joining of the divided parts (21) and (22) in this case is also accomplished by engaging engaging parts (24a) and (24b) provided for divided member (21) and engagement recesses (28a) and (28b) provided in divided member (22), and this joining will be reliable, since divided member (21) has a restorative force produced by elasticity.

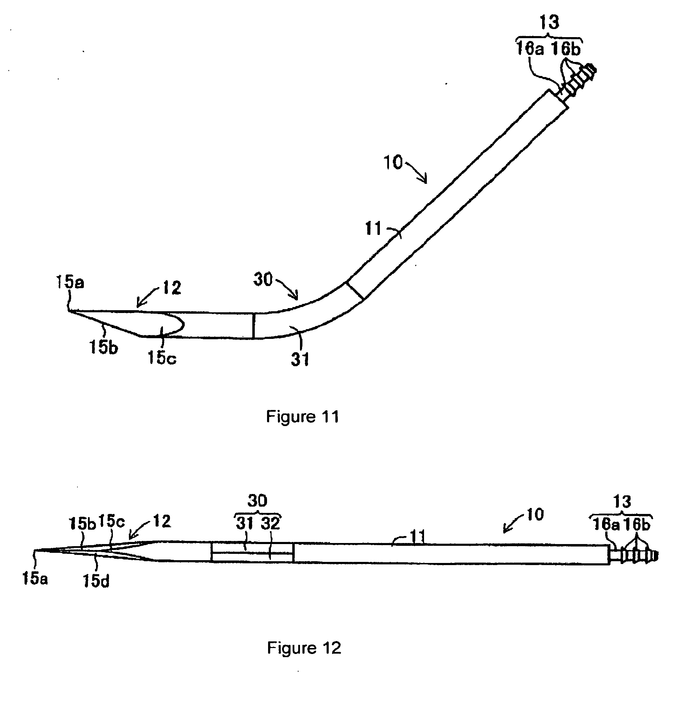

Figures 11 and 12 show medical needle (10) to which reinforcing tool (30) pertaining to a second embodiment of the present invention is attached. Medical needle (10) is used while narrow diameter part (14) is bent, and reinforcing tool (30) is used after being bent beforehand to the angle at which narrow diameter part (14) is bent. Reinforcing tool (30) is constituted by detachably joining two divided parts (31) and (32) arranged at the right and left. Divided member (31) is such that when divided member (21) of the abovementioned reinforcing tool (20) is bent, one longitudinal edge will be at the inside periphery and the other edge will be at the outside periphery, and divided member (32) is such that when divided member (22) of the abovementioned reinforcing tool (20) is bent, in one longitudinal edge will be at the inside periphery and the other edge will be at the outside periphery

The constitution of the other portions of reinforcing tool (30) is the same as for said reinforcing tool (20) in the first embodiment. Therefore, divided member (31) can be flexed toward the central axis and attached to or detached from divided member (32) by pressing the two edges of divided member (31) toward the middle. Medical needle (10) can therefore be bent to penetrate the patient's wound easily, and the strength of narrow diameter part (14) of medical needle (10) can in this case be increased. Otherwise, the function and effects of reinforcing tool (30) pertaining to this embodiment are the same as for said reinforcing tool (20) in the first embodiment. Also, in this embodiment a plurality of reinforcing tools with different bending angles can be prepared, so that a reinforcing tool suitably corresponding to the bending angle of medical needle (10) can be used.

The present invention is also not limited to the abovementioned embodiments and can be implemented with appropriate modifications. For example, in the abovementioned embodiments, reinforcing tools (20) and (30) are constructed from stainless steel, but the materials to construct reinforcing tools (20) and (30) could be reinforced plastic or another material with the prescribed strength, rather than stainless steel. Also, in the abovementioned embodiments, reinforcing tools (20) and (30) are to be removed when medical needle (10) is pulled out of the patient's body, but medical needle (10) can also be pulled out with reinforcing tools (20) and (30) attached by providing slip-preventing roughness to the outer peripheral surfaces of reinforcing tools (20) and (30).

In addition, slipping can also be prevented by providing a level difference or gap between the outer peripheral surface of medical needle (10) and the outer peripheral surface of reinforcing tool (20) or (30). A notched groove (25) is also provided in divided parts (21) and (31), but if divided parts (21) and (31) are constructed so that they can be flexed even without notched groove (25), notched groove (25) need not be provided. In addition, the abovementioned reinforcing tool (20) in the first embodiment is made so that divided parts (21) and (22) are arranged above and below medical needle (10), but the placement of divided parts (21) and (22) in this case could be in any direction around the axis of medical needle (10), they could be reversed top and bottom, or they could be arranged to the left and right.

Reinforcing tools (20) and (30) can also be constructed from a material that can bend. By so doing, reinforcing tools (20) and (30) can be bent to match the bending angle of narrow diameter part (14) when narrow diameter part (14) of medical needle (10) is bent to a prescribed angle. It is preferred for reinforcing tools (20) and (30) in this case to be constructed with a material of greater hardness than that of narrow diameter part (14), and that is harder to bend than narrow diameter part (14) of medical needle (10). They can then be bent to match the bending angle of narrow diameter part (14), but narrow diameter part (14) and reinforcing tool (20) or reinforcing tool (30) will not bend due to the combined strength of narrow diameter part (14) and reinforcing tool (20) or (30). Because of this, medical needle (10) can be bent to a prescribed angle [if needed], while during the actual operation medical needle (10) can be prevented from bending.

## Claims

1. A tool (20, 30) for reinforcing a medical needle (10) having a recessed portion (14) of relatively narrow diameter and main body portions of relatively larger diameter on either side of said recessed portion (14) wherein the tool comprises a tubular cylindrical body, **characterized in that** the tubular cylindrical body has two separable halves (21,22) which are detachably attachable around said recessed portion (14) of the medical needle (10).

2. The tool according to Claim 1 wherein the body (20,30) of the tool has an outer diameter which is substantially the same size as an outer diameter of the main body portions of the medical needle.

3. The tool according to Claim 1 wherein the cylindrical body comprises two parts (21, 22, 31, 32) joinable along longitudinal edges thereof.

4. The tool according to Claim 3 wherein said parts (21, 22, 31, 32) include mutually cooperative engaging means (24a, 24b; 28a, 28b) and said engaging means are releasably attachable together.

5. The tool according to Claim 1 wherein the tool (20, 30) is bendable.

6. The tool according to any one of Claims 1-5 wherein said medical needle (11) may be bent to form a prescribed angle and wherein said tool is bendable in advance of attachment to said medical needle to match said prescribed angle.

7. A medical instrument comprising a medical needle (10) having a recessed portion (14) of relatively narrow diameter and main body portions of relatively larger diameter on either side of said recessed portion (14) and a tool (20, 30) for reinforcing said medical needle wherein the tool comprises a tubular cylindrical body, **characterized in that** the tubular cylindrical body has two separable halves (21,22) which are detachably attachable around said recessed portion (14) of the medical needle (10).

8. The medical instrument according to Claim 7 wherein the body of the tool has an outer diameter which is substantially the same size as an outer diameter of the main body portions of the medical needle.

9. The medical instrument according to Claim 7 wherein the cylindrical body of the tool comprises two parts (21, 22, 31, 32) joinable along longitudinal edges thereof.

10. The medical instrument according to Claim 9 wherein said parts (21, 22, 31, 32) include mutually cooperative engaging means (24a, 24b; 28a, 28b) and said engaging means are releasably attachable together.

11. The medical instrument according to Claim 7, wherein the tool (20, 30) is bendable.

12. The medical instrument according to any one of Claims 7-11 wherein said medical needle (11) may be bent to form a prescribed angle and wherein said tool is bendable in advance of attachment to said medical needle to match said prescribed angle.

## Patentansprüche

1. Werkzeug (20, 30) zur Verstärkung einer medizinischen Nadel (10) mit einem ausgesparten Teil (14) mit einem relativ schmalen Durchmesser und Hauptkörperteilen mit relativ großem Durchmesser auf beiden Seiten des ausgesparten Teils (14), wobei das Werkzeug einen röhrenförmigen zylindrischen Körper umfasst, **dadurch gekennzeichnet, dass** der röhrenförmige zylindrische Körper zwei trennbare Hälften (21, 22) aufweist, die lösbar um den ausgesparten Teil (14) der medizinischen Nadel (10) herum befestigt werden können.

2. Werkzeug nach Anspruch 1, wobei der Körper des Werkzeugs (20, 30) einen Außendurchmesser aufweist, der im Wesentlichen die gleiche Größe aufweist wie der Außendurchmesser der Hauptkörperteile der medizinischen Nadel.

3. Werkzeug nach Anspruch 1, wobei der zylindrische Körper zwei Teile (21, 22, 31, 32) umfasst, die entlang Längsrändern davon verbunden werden können.

4. Werkzeug nach Anspruch 3, wobei die Teile (21, 22, 31, 32) zusammenwirkende Eingriffsmittel (24a, 24b; 28a, 28b) enthalten und die Eingriffsmittel lösbar aneinander befestigt werden können.

5. Werkzeug nach Anspruch 1, wobei das Werkzeug (20, 30) biegbar ist.

6. Werkzeug nach einem der Ansprüche 1 - 5, wobei die medizinische Nadel (11) zur Bildung eines vorgeschriebenen Winkels gebogen werden kann und wobei das Werkzeug vor der Befestigung an der medizinischen Nadel gebogen werden kann, um mit dem vorgeschriebenen Winkel zusammenzupassen.

7. Medizinisches Gerät, das eine medizinische Nadel (10) mit einem ausgesparten Teil (14) mit einem relativ schmalen Durchmesser und Hauptkörperteilen mit relativ großem Durchmesser auf beiden Seiten des ausgesparten Teils (14) und ein Werkzeug (20, 30) zur Verstärkung der medizinischen Nadel umfasst, wobei das Werkzeug einen röhrenförmigen zylindrischen Körper umfasst, **dadurch gekennzeichnet, dass** der röhrenförmige zylindrische Körper zwei trennbare Hälften (21, 22) aufweist, die lösbar um den ausgesparten Teil (14) der medizinische Nadel (10) herum befestigt werden können.

8. Medizinisches Gerät nach Anspruch 7, wobei der Körper des Werkzeugs einen Außendurchmesser aufweist, der im Wesentlichen die gleiche Größe aufweist wie der Außendurchmesser der Hauptkörperteile der medizinischen Nadel.

9. Medizinisches Gerät nach Anspruch 7, wobei der zylindrische Körper des Werkzeugs zwei Teile (21, 22, 31, 32) umfasst, die entlang Längsrändern davon verbunden werden können.

10. Medizinisches Gerät nach Anspruch 9, wobei die Teile (21, 22, 31, 32) zusammenwirkende Eingriffsmittel (24a, 24b; 28a, 28b) enthalten und die Eingriffsmittel lösbar aneinander befestigt werden können.

11. Medizinisches Gerät nach Anspruch 7, wobei das Werkzeug (20, 30) biegbar ist.

12. Medizinisches Gerät nach einem der Ansprüche 7-11, wobei die medizinische Nadel (11) zur Bildung eines vorgeschriebenen Winkels gebogen werden kann und wobei das Werkzeug vor der Befestigung an der medizinischen Nadel gebogen werden kann, um mit dem vorgeschriebenen Winkel zusammenzupassen.

## Revendications

1. Outil (20, 30) pour renforcer une aiguille (10) médicale munie d'une portion (14) renfoncée d'un diamètre relativement étroit et de portions de corps principal d'un diamètre relativement grand de chaque côté de ladite portion (14) renfoncée, ledit outil comportant un corps tubulaire cylindrique, **caractérisé en ce que** le corps tubulaire cylindrique a deux moitiés (21, 22) séparables qui sont fixées de manière détachable autour de ladite portion (14) renfoncée de l'aiguille (10) médicale.

2. Outil selon la revendication 1 dans lequel le corps de l'outil (20, 30) possède un diamètre externe qui est sensiblement de la même taille qu'un diamètre externe des portions de corps principal de l'aiguille médicale.

3. Outil selon la revendication 1 dans lequel le corps cylindrique comporte deux parties (21, 22, 31, 32) pouvant être assemblées le long de leurs bords longitudinaux.

4. Outil selon la revendication 3 dans lequel lesdites parties (21, 22, 31, 32) comprennent des moyens (24a, 24b ; 28a, 28b) de prise à interaction mutuelle et lesdits moyens de prise sont fixés ensemble d'une manière libérable.

5. Outil selon la revendication 1, ledit outil (20, 30) étant pliable.

6. Outil selon l'une quelconque des revendications 1 à 5, ladite aiguille (11) médicale pouvant être pliée pour former un angle prescrit et ledit outil étant pliable avant d'être fixé à ladite aiguille médicale pour correspondre audit angle prescrit.

7. Instrument médical comportant une aiguille (10) médicale possédant une portion (14) renfoncée d'un diamètre relativement étroit et des portions de corps principal d'un diamètre relativement grand de chaque côté de ladite portion (14) renfoncée et un outil (20, 30) pour renforcer ladite aiguille médicale, l'outil comportant un corps tubulaire cylindrique, **caractérisé en ce que** le corps tubulaire cylindrique a deux moitiés (21, 22) séparables qui sont fixées de manière détachable autour de ladite portion (14) renfoncée de l'aiguille (10) médicale.

8. Instrument médical selon la revendication 7 dans lequel le corps de l'outil possède un diamètre externe qui est sensiblement de la même taille qu'un diamètre externe des portions de corps principal de l'aiguille médicale.

9. Instrument médical selon la revendication 7 dans lequel le corps cylindrique de l'outil comporte deux parties (21, 22, 31, 32) pouvant être assemblées le long de leurs bords longitudinaux.

10. Instrument médical selon la revendication 9 dans lequel lesdites parties (21, 22, 31, 32) comprennent des moyens (24a, 24b ; 28a, 28b) de prise à interaction mutuelle et lesdits moyens de prise peuvent être fixés ensemble de manière libérable.

11. Instrument médical selon la revendication 7, dans lequel l'outil (20, 30) est pliable.

12. Instrument médical selon l'une quelconque des revendications 7 à 11 dans lequel ladite aiguille (11) médicale peut être pliée pour former un angle prescrit et dans lequel ledit outil est pliable avant d'être fixé à ladite aiguille médicale pour correspondre audit angle prescrit.
